# EUROPÄISCHE PATENTANMELDUNG

(11) **EP 4 851 296 A1**
(43) Veröffentlichungstag der Anmeldung: **09.12.2026**
(21) Anmeldenummer: 25181513.0
(22) Anmeldetag: 06.06.2025
(51) Int. Cl.: C12P 7/26, C12N 9/04, C12P 7/42

(54) **VERFAHREN ZUR HERSTELLUNG VON 2-KETO-D-GLUCONAT**

(71) Anmelder: Annikki GmbH, 8074 Raaba-Grambach (AT)
(72) Erfinder: Staunig, Nicole, 8074 Raaba-Grambach (AT)
(74) Vertreter: Schwarz & Partner Patentanwälte GmbH

(57) **Zusammenfassung**

Verfahren zur Herstellung von 2-Keto-D-Gluconat durch Oxidation von D-Glucose, welche in einer wässrigen Lösung vorliegt, gekennzeichnet durch die Kombination der Maßnahmen,
dass die D-Glucose *in vitro* durch Behandeln mit einer NAD(P)⁺-abhängigen Glucose-Dehydrogenase und einer NAD(P)⁺-abhängigen Gluconat-2-Dehydrogenase zu 2-Keto-D-Gluconat oxidiert wird, wobei reduzierter Kofaktor NAD(P)H gebildet wird, und
dass der reduzierte Kofaktor NAD(P)H enzymatisch mit einer NAD(P)H-Oxidase und/oder einer NAD(P)H-abhängigen Alkoholdehydrogenase zu NAD(P)⁺ oxidiert wird, wobei Sauerstoff und/oder eine Keto-Verbindung als Kosubstrat für die Oxidation des reduzierten Kofaktors eingesetzt wird.

## Beschreibung

Die Erfindung betrifft ein Verfahren zur Herstellung von 2-Keto-D-Gluconat aus D-Glucose.

### Hintergrund der Erfindung

2-Keto-D-Gluconsäure/2-Keto-D-Gluconat (2KGA), ein Oxidationsprodukt der D-Gluconsäure (D-Gluconat), kann zur Herstellung von Isoascorbinsäure (Isomer von Vitamin C) und als chiraler Building Block zur Synthese von diversen (potenziell biologisch wirksamen) heterozyklischen Verbindungen eingesetzt werden (Stottmeister et al., 2005).

2KGA kann beispielsweise chemisch aus D-Fructose hergestellt werden, welche zunächst mit Aceton geschützt wird. Die Oxidation selbst wird mit Kaliumpermanganat bewerkstelligt. Nach der Entfernung der Aceton-Schutzgruppen mit methanolischer HCl wird der Methylester von 2KGA erhalten. Hierbei werden allerdings (im Labormaßstab) nur Ausbeuten von 40% erreicht, was für eine industrielle Anwendbarkeit problematisch ist (Stottmeister et al., 2005).

Alternativ zum beschriebenen chemischen Herstellungsverfahren für 2KGA sind bereits biotechnologische Verfahren etabliert, die überwiegend fermentativ durchgeführt werden (Kataoka, 2024).

In der industriellen Produktion von 2KGA werden beispielsweise *Pseudomonas*-Stämme eingesetzt, welche jedoch anfällig für Phagen-Kontamination sind, die sich negativ auf die Produktivität auswirkt. Zeng et al. (2019) nutzten daher einen engineerten *Gluconobacter japonicus*-Stamm zur Oxidation von D-Glucose zu 2KGA, wobei bei einem Fed-Batch-Verfahren in einem 15 Liter-Reaktor ein Titer von 239,4 g/l und eine Ausbeute von 92,5% in 80 h erreicht werden konnten.

Li et al. (2016) verwendeten einen *Gluconobacter oxydans*-Stamm mit überexprimierter membrangebundener Gluconat-2-Dehydrogenase zur Umwandlung von D-Glucose (270 g/l) zu 2KGA (321 g/l) in 18 h (Ausbeute 99,1 %; Produktivität 17,8 g/(l·h)).

Sun et al. (2020) verwendeten Reisstroh-Hydrolysat als Substrat für ein fermentatives zweistufiges semikontinuierliches Verfahren mittels *Pseudomonas plecoglossicida* JUIM01, wobei insgesamt 50 kg 2KGA aus 50,7 kg D-Glucose (0,9867 g/g) erhalten wurden.

Die Oxidation von D-Glucose zu 2KGA mittels Essigsäurebakterien wie *Gluconobacter* sp. wird durch membrangebundene Dehydrogenasen katalysiert. Die membrangebundene D-Glucose-Dehydrogenase (mGDH), ein Pyrrolochinolinchinon-(PQQ-)abhängiges Enzym, oxidiert die D-Glucose zunächst zu D-Gluconat, welches in weiterer Folge von der D-Gluconat-Dehydrogenase (GADH), einem Flavoprotein-Cytochrom C-Komplex, zu 2-Keto-D-Gluconat oxidiert wird. Beide Enzyme sind an die Atmungskette angebunden (Kataoka, 2024).

Darüber hinaus ist in Essigsäure-Bakterien (z.B. *Acetobacter-* und *Gluconobacter*-Spezies) die cytosolische 2-Keto-Gluconat-Reduktase/Gluconat-2-Dehydrogenase (EC 1.1.1.215) bekannt, welche ebenfalls die Oxidation von D-Gluconat zu 2-Keto-D-Gluconat (sowie die Rückreaktion) katalysiert (Chiyonobu et al., 1973; Chiyonobu et al., 1976; Adachi et al., 1978; Saichana et al., 2007).

In dem Übersichtsartikel von Luo et al. (2021) werden chemische und biokatalytische Routen zu 2-Keto-D-Gluconat vorgestellt. Dabei schreiben die Autoren, dass die fermentative Herstellung zum Zeitpunkt des Verfassens des Artikels die effizienteste Methode sei.

Hier setzt nun die folgende Erfindung an und möchte ein verbessertes Verfahren zur Herstellung von 2-Keto-D-Gluconat zur Verfügung stellen.

### Detaillierte Beschreibung der Erfindung

Die Aufgabe zur Herstellung von 2-Keto-D-Gluconat aus D-Glucose wird in einem Verfahren gelöst, welches gekennzeichnet ist durch die Kombination der Maßnahmen, dass die D-Glucose *in vitro* durch Behandeln mit einer NAD(P)'-abhängigen Glucose-Dehydrogenase und einer NAD(P)'-abhängigen Gluconat-2-Dehydrogenase zu 2-Keto-D-Gluconat oxidiert wird, wobei reduzierter Kofaktor NAD(P)H gebildet wird, und dass der reduzierte Kofaktor NAD(P)H enzymatisch mit einer NAD(P)H-Oxidase und/oder einer NAD(P)H-abhängigen Alkoholdehydrogenase zu NAD(P)⁺ oxidiert wird, wobei Sauerstoff und/oder eine Keto-Verbindung als Kosubstrat für die Oxidation des reduzierten Kofaktors eingesetzt wird.

"Keto-Verbindung" bezeichnet in diesem Zusammenhang eine organische Verbindung mit einer Keto-Gruppe, also z. B. Aldehyde und Ketone.

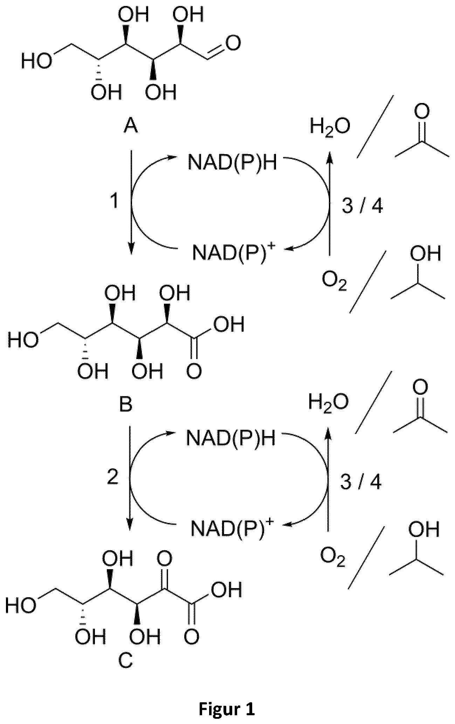

Die Figur 1 zeigt die eine bevorzugte Ausführungsform des erfindungsgemäßen Verfahrens. Die Bezeichnung A steht für D-Glucose, B für D-Gluconsäure/D-Gluconat, C für 2-Keto-D-gluconsäure/2-Keto-D-gluconat, 1 für Glucose-Dehydrogenase, 2 für Gluconat-2-Dehydrogenase, 3 für NAD(P)H-Oxidase und 4 für Alkoholdehydrogenase.

Es hat sich überraschenderweise gezeigt, dass das erfindungsgemäße Verfahren zur Herstellung von 2-Keto-D-Gluconat als Eintopf-Verfahren durchgeführt werden kann, das bedeutet, dass alle Reaktionsschritte im selben Reaktionsgefäß (Reaktor) durchgeführt werden können.

Bevorzugt werden eine einzige NAD(P)H-Oxidase und/oder eine einzige NAD(P)H-abhängigen Alkoholdehydrogenase zur Oxidation des reduzierten Kofaktors NAD(P)H eingesetzt.

Besonders bevorzugt wird eine einzige NAD(P)H-Oxidase zur Oxidation des reduzierten Kofaktors NAD(P)H eingesetzt.

In einer besonders bevorzugten Ausführungsform des erfindungsgemäßen Verfahrens ist die Keto-Verbindung Aceton.

Besonders bevorzugt wird das vorliegende Verfahren außerhalb eines Organismus durchgeführt (*in vitro*), das heißt nicht in einer ganzen Zelle und auch nicht fermentativ.

Besonders bevorzugt wird das Verfahren in einer einzigen flüssigen Phase durchgeführt.

In weiteren bevorzugten Ausführungsform des erfindungsgemäßen Verfahrens beträgt die Konzentration der Substrate in der wässerigen Lösung zwischen 5 und 250 g/l.

Der besonders bevorzugte Temperaturbereich der Reaktionen liegt zwischen 15 und 50 °C.

Der besonders bevorzugte pH-Bereich der Reaktionen liegt zwischen pH 6 und 10,5.

Besonders bevorzugt wird das vorliegende Verfahren außerhalb eines Organismus durchgeführt (*in vitro*), das heißt nicht in einer ganzen Zelle und auch nicht fermentativ.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind. Zudem können die Enzyme auch in lyophilisierter oder sprühgetrockneter oder immobilisierter Form im erfindungsgemäßen Verfahren eingesetzt werden. Die erfindungsgemäß eingesetzten Enzyme können auch in einer im Wesentlichen gereinigten Form verwendet werden, so dass die Enzympräparate keine oder im Wesentlichen keine Bestandteile umfassen, die von den Zellen bzw. der Kultur, in der diese exprimiert wurden, stammen. Die beispielsweise durch rekombinante Expression (z.B. in *E. coli*) hergestellten Enzyme können durch Verfahren, die im Stand der Technik bekannt sind, gereinigt werden, um nach u.a. Ultrafiltration und DNA-Entfernung auch in und zur Herstellung von Lebensmitteln eingesetzt zu werden.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

Mit den nachfolgenden Beispielen werden bevorzugte Ausführungsformen der Erfindung noch näher beschrieben.

### Materialien

Triethanolamin und Natriumdodecylsulfat (SDS) wurden von Carl Roth, 2-Propanol wurde von Honeywell, IPTG (Isopropyl-β-D-thiogalactopyranosid), D-Glucose, Natrium-D-Gluconat und Hemicalcium-2-Keto-D-gluconat-Hydrat wurden von Sigma-Aldrich und NAD⁺, NADH-Dinatriumsalz, NADP⁺-Dinatriumsalz, NADPH-Tetranatriumsalz sowie Acetonitril wurde von PanReac AppliChem (ITW Reagents) bezogen.

### Produktion der Enzyme & Herstellung der Lysate

### Allgemeines zur Expression von rekombinanten Enzymen in E. coli

Für die rekombinante Enzymproduktion in einem *Escherichia* coli-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und HindIII wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E*. *coli*-Zellen Top10F' transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optischen Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymtypen und Spenderorganismen für die in den Beispielen verwendeten Enzyme.**

| **Enzymtyp (EC-Klasse)** | **katalysierte Reaktion** | **Spenderorganismus** | **Sequenzen/Literatur** |
|---|---|---|---|
| Glucose-Dehydrogenase I (GDH I) | D-Glucose → D-Gluconat | *Bacillus spizizenii* | (NCBI Protein Database: WP_003225027.1) |
| Glucose-Dehydrogenase II (GDH II) | | *Priestia megaterium* | (NCBI Protein Database: BAA01476.1) |
| Gluconat-2-Dehydogenase | D-Gluconat → 2-Keto-D-Gluconat | *Gluconobacter frateurii* | (NCBI Protein Database: WP_099182896.1) |
| NAD(P)H-Oxidase | NAD(P)H → NAD(P)⁺ | *Carnobacterium divergens,* Mutante | WO 2025/012236 A1 |
| Alkoholdehydrogenase | Aceton → 2-Propanol | *Thermoanaerobacter brockii* | (NCBI Protein Database: CAA46053.1) |

### Analytische Methoden

### High Performance Liquid Chromatography (HPLC)

Zur Quantifizierung von D-Glucose mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RCM-Monosaccharide Ca+2 (8%) Säule mit entsprechender Vorsäule verwendet und mit 3,5% (v/v) 2-Propanol in Reinstwasser isokratisch eluiert.

### High Performance Anion Exchange Chromatography (HPAEC)

Zur Quantifizierung von D-Gluconat und 2-Keto-D-Gluconat mittels HPAEC (High Performance Anion Exchange Chromatography) wurde ein Dionex ICS6000 System mit AS-AP Autosampler verwendet. Die Messung erfolgte mittels Leitfähigkeitsdetektion (CD) gekoppelt an einen Dionex AERS 500 elektrolytisch regenerierten Suppressor im externen Wassermodus. Zur Trennung der Analyten wurde eine Dionex lonPac AS11-HC-4µm Säule mit entsprechender Vorsäule sowie einem NaOH-Gradienten verwendet. Das Laufmittel wurde zusätzlich mit einer Dionex ATC (Anion Trap Column) vorbehandelt.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung oder der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Oxidation von D-Glucose zu 2-Keto-D-Gluconat

In vier 2 ml Glas-Vials wurden folgende Komponenten vermischt: 200 µl eines 500 mM TEA-HCl-Puffers (pH 8,5), 100 µl einer 278 mM D-Glucose-Lösung, 10 µl Glucose-Dehydrogenase-Lysat (siehe nachfolgende Tabelle 2), 10 µl Oxidoreduktase-Lysat, 5 µl einer 5 mM NADP⁺-Lösung (finale Konzentration 0,05 mM) sowie 10 U NAD(P)H-Oxidase-Lysat oder 10 U Alkoholdehydrogenase I-Lysat. Alle Ansätze wurden mit deionisiertem Wasser auf 500 µl Gesamtvolumen aufgefüllt. Zusätzlich enthielten jene Ansätze mit Alkoholdehydrogenase 15 µl Aceton.

Die Ansätze wurden 20 h bei 30 °C unter kontinuierlichem Schütteln unter atmosphärischer Luft (1200 rpm, Eppendorf Thermomixer) inkubiert.

Zur Aufarbeitung wurden 100 µl eines Ansatzes mit 200 µl Acetonitril versetzt und bei 60 °C für 20 min inkubiert (1200 rpm, Eppendorf Thermomixer). Nach der Denaturierung wurde die Probe mit 700 µl Reinstwasser verdünnt und im Anschluss 10 min bei max. g zentrifugiert. 150 µl des klaren Überstands wurden in ein HPLC-Vial überführt und vermessen (HPLC, RI-Detektion). Für die Quantifizierung der Zuckersäuren wurde der Überstand 1:40 mit Reinstwasser verdünnt und auf der HPAEC (Leitfähigkeitsdetektion) gemessen.

Die Ergebnisse sind der nachfolgenden Tabelle 2 dargestellt.

**Tabelle 2**

| **Kofaktor-Regeneration** | **Glucose-Dehydrogenase** | **Ausbeute 2-Keto-D-Gluconat [%]** |
|---|---|---|
| NAD(P)H-Oxidase | GDH I | 96 |

| | | |
|---|---|---|
| | GDH II | 96 |
| Alkoholdehydrogenase / Aceton | GDH I | 9 |
| | GDH II | 9 |

### Beispiel 2

### Oxidation von D-Gluconat zu 2-Keto-D-Gluconat

In zwei 2 ml Glas-Vials wurden folgende Komponenten vermischt: 200 µl eines 500 mM TEA-HCl-Puffers (pH 8,5), 100 µl einer 229 mM Natrium-D-Gluconat-Lösung, 10 µl Oxidoreduktase-Lysat, 5 µl einer 5 mM NADP⁺-Lösung (finale Konzentration 0,05 mM) sowie 10 U NAD(P)H-Oxidase-Lysat oder 10 U Alkoholdehydrogenase-Lysat (siehe nachfolgende Tabelle 3). Alle Ansätze wurden mit deionisiertem Wasser auf 500 µl Gesamtvolumen aufgefüllt. Zusätzlich enthielten jene Ansätze mit Alkoholdehydrogenase 15 µl Aceton.

Die Ansätze wurden 20 h bei 30 °C unter kontinuierlichem Schütteln unter atmosphärischer Luft (1200 rpm, Eppendorf Thermomixer) inkubiert.

Zur Aufarbeitung wurden 100 µl eines Ansatzes mit 200 µl Methanol versetzt und bei 60 °C für 20 min inkubiert (1200 rpm, Eppendorf Thermomixer). Nach der Denaturierung wurde die Probe mit 700 µl Reinstwasser verdünnt und im Anschluss 10 min bei max. g zentrifugiert. Der Überstand wurde 1:40 mit Reinstwasser verdünnt und auf der HPAEC (Leitfähigkeitsdetektion) gemessen.

Die Ergebnisse sind der nachfolgenden Tabelle 3 dargestellt.

**Tabelle 3**

| **Kofaktor-Regeneration** | **Ausbeute 5-Keto-D-Gluconat [%]** |
|---|---|
| NAD(P)H-Oxidase | 97 |
| Alkoholdehydrogenase / Aceton | 27 |

### Literatur

Stottmeister, U., Aurich, A., Wilde, H., Andersch, J., Schmidt, S., & Sicker, D. (2005). White biotechnology for green chemistry: fermentative 2-oxocarboxylic acids as novel building blocks for subsequent chemical syntheses. Journal of Industrial Microbiology and Biotechnology, 32(11-12), 651-664. https://doi.org/10.1007/s10295-005-0254-x
Kataoka, N. (2024). Ketogluconate production by Gluconobacter strains: enzymes and biotechnological applications. Bioscience, Biotechnology, and Biochemistry, 88(5), 499-508. https://doi.org/10.1093/bbb/zbae013
Zeng, W., Cai, W., Liu, L., Du, G., Chen, J., & Zhou, J. (2019). Efficient biosynthesis of 2-keto-D-gluconic acid by fed-batch culture of metabolically engineered Gluconobacter japonicus. Synthetic and Systems Biotechnology, 4(3), 134-141. https://doi.org/10.1016/j.synbio.2019.07.001
Li, K., Mao, X., Liu, L., Lin, J., Sun, M., Wie, D., & Yang, S. (2016). Overexpression of membrane-bound gluconate-2-dehydrogenase to enhance the production of 2-keto-D-gluconic acid by Gluconobacter oxydans. Microbial Cell Factories, 15, 121. https://doi.org/10.1186/s12934-016-0521-8
Sun, L., Wang, D.-M., Sun, W.-J., Cui, F.-J., Gong, J.-So., Zhang, X.-M., Shi, J.-S., & Xu, Z.-H. (2020). Two-Stage Semi-Continuous 2-Keto-Gluconic Acid (2KGA) Production by Pseudomonas plecoglossicida JUIM01 From Rice Starch Hydrolyzate. Frontiers in Bioengineering and Biotechnology, 8, 120. https://doi.org/10.3389/fbioe.2020.00120
Chiyonobu, T., Adachi, O., & Ameyama, M. (1973). Purification and Properties of 2-Ketogluconate Reductase from Gluconobacter liquefaciens. Agricultural and Biological Chemistry, 37(12), 2871-2878. https://doi.org/10.1080/00021369.1973.10861083
Chiyonobu, T., Shinagawa, E., Adachi, O., & Ameyama, M. (1976). Purification, Crystallization and Properties of 2-Ketogluconate Reductase from Acetobacter rancens. Agricultural and Biological Chemistry, 40(1), 175-184. https://doi.org/10.1080/00021369.1976.10862016
Adachi, O., Chiyonobu, T., Shinagawa, E., Matsushita, K., & Ameyama, M. (1978). Crystalline 2-Ketogluconate Reductase from Acetobacter ascendens, the Second Instance of Crystalline Enzyme in Genus Acetobacter. Agricultural and Biological Chemistry, 42(11), 2057-2062. https://doi.org/10.1080/00021369.1978.10863307
Saichana, I., Ano, Y., Adachi, O., Matsushita, K., & Toyama, H. (2007). Preparation of Enzymes Required for Enzymatic Quantification of 5-Keto-D-gluconate and 2-Keto-D-gluconate. Bioscience, Biotechnology, and Biochemistry, 71(10), 2478-2486. https://doi.org/10.1271/bbb.70259
Luo, Z., Yu, S., Zeng, W., & Zhou, J. (2021). Comparative analysis of the chemical and biochemical synthesis of keto acids. Biotechnology Advances, 47, 107706. https://doi.org/10.1016/j.biotechadv.2021.107706
Lin, B., & Tao, Y. (2017). Whole-cell biocatalysts by design. Microbial Cell Factories, 16, 106. https://doi.org/10.1186/s12934-017-0724-7
Protein [Internet]. Bethesda (MD): National Library of Medicine (US), National Center for Biotechnology Information. Accession No. WP_003225027.1, glucose 1-dehydrogenase [Bacillus spizizenii]. Verfügbar unter: https://www.ncbi.nlm.nih.gov/protein/WP_003225027.1
Protein [Internet]. Bethesda (MD): National Library of Medicine (US), National Center for Biotechnology Information. Accession No. BAA01476.1, glucose dehydrogenase [Priestia megaterium]. Verfügbar unter: https://www.ncbi.nlm.nih.gov/protein/BAA01476.1
Protein [Internet]. Bethesda (MD): National Library of Medicine (US), National Center for Biotechnology Information. Accession No. WP_099182896.1, 2-hydroxyacid dehydrogenase [Gluconobacter frateurii]. Verfügbar unter: https://www.ncbi.nlm.nih.gov/protein/WP_099182896.1
Protein [Internet]. Bethesda (MD): National Library of Medicine (US), National Center for Biotechnology Information. Accession No. CAA46053.1, alcohol dehydrogenase [Thermoanaerobacter brockii]. Verfügbar unter: https://www.ncbi.nlm.nih.gov/protein/CAA46053.1

## Patentansprüche

1. Verfahren zur Herstellung von 2-Keto-D-Gluconat durch Oxidation von D-Glucose, welche in einer wässrigen Lösung vorliegt, **gekennzeichnet durch** die Kombination der Maßnahmen,
dass die D-Glucose *in vitro* durch Behandeln mit einer NAD(P)⁺-abhängigen Glucose-Dehydrogenase und einer NAD(P)⁺-abhängigen Gluconat-2-Dehydrogenase zu 2-Keto-D-Gluconat oxidiert wird, wobei reduzierter Kofaktor NAD(P)H gebildet wird, und
dass der reduzierte Kofaktor NAD(P)H enzymatisch mit einer NAD(P)H-Oxidase und/oder einer NAD(P)H-abhängigen Alkoholdehydrogenase zu NAD(P)⁺ oxidiert wird, wobei Sauerstoff und/oder eine Keto-Verbindung als Kosubstrat für die Oxidation des reduzierten Kofaktors eingesetzt wird.

2. Verfahren nach Anspruch 1, **dadurch gekennzeichnet, dass** das Verfahren als Eintopf-Verfahren durchgeführt wird.

3. Verfahren nach einem der Ansprüche 1 oder 2, **dadurch gekennzeichnet, dass** eine einzige NAD(P)H-Oxidase und/oder eine einzige NAD(P)H-abhängigen Alkoholdehydrogenase eingesetzt wird.

4. Verfahren nach einem der Ansprüche 1 bis 3, **dadurch gekennzeichnet, dass** eine einzige NAD(P)H-Oxidase eingesetzt wird.

5. Verfahren nach einem der Ansprüche 1 bis 3, **dadurch gekennzeichnet, dass** die Keto-Verbindung Aceton ist.

6. Verfahren nach einem der Ansprüche 1 bis 5, **dadurch gekennzeichnet, dass** das Verfahren in *vitro* durchgeführt wird.

7. Verfahren nach einem der Ansprüche 1 bis 6, **dadurch gekennzeichnet, dass** es in einer einzigen flüssigen Phase durchgeführt wird.
